(19) Europäisches Patentamt
European Patent Office
Office européen des brevets

(11) Veröffentlichungsnummer: **0 170 831**
**B1**

(12) # EUROPÄISCHE PATENTSCHRIFT

(45) Veröffentlichungstag der Patentschrift: **28.12.88**

(51) Int. Cl.4: **C 07 D 233/60**, C 07 D 249/08, A 01 N 43/653, A 01 N 43/50

(21) Anmeldenummer: **85107312.2**

(22) Anmeldetag: **13.06.85**

(54) **Azolylether, Verfahren zur Herstellung und ihre Verwendung als Fungizide.**

(30) Priorität: **19.06.84 DE 3422611**

(43) Veröffentlichungstag der Anmeldung: **12.02.86 Patentblatt 86/7**

(45) Bekanntmachung des Hinweises auf die Patenterteilung: **28.12.88 Patentblatt 88/52**

(84) Benannte Vertragsstaaten: **AT BE CH DE FR GB IT LI NL SE**

(56) Entgegenhaltungen:
**EP-A- 0 021 327**
**EP-A- 0 028 363**
**EP-A- 0 129 186**
**US-A- 4 045 568**

(73) Patentinhaber: **BASF Aktiengesellschaft, Carl-Bosch-Strasse 38, D-6700 Ludwigshafen (DE)**

(72) Erfinder: **Tuerk, Wolfgang, Dr., Wittelsbachstrasse 61, D-6700 Ludwigshafen (DE)**
Erfinder: **Buschmann, Ernst, Dr., Georg-Ludwig-Krebs-Strasse 10, D-6700 Ludwigshafen (DE)**
Erfinder: **Sproesser, Linhard, Dr., Goethestrasse 4, D-6702 Bad Duerkheim (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

EP 0 170 831 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neuen Azolylether, Verfahren zu ihrer Herstellung, deren Verwendung als Fungizide, Fungizide Mischungen, die diese Wirkstoffe enthalten, Verfahren zur Herstellung solcher fungizider Mischungen sowie Verfahren zur Bekämpfung von Schadpilzen mit diesen Wirkstoffen.

Es ist bekannt, Azolylverbindungen, z.B. das 1-(2′-(2,4-Dichlorphenyl)-2′-(2-propenyloxy)-ethyl-1H-imidazol als Fungizid zu verwenden (GB-A 1 318 590).

Seine Wirkung ist jedoch nicht in allen Anwendungsbereichen, insbesondere bei niedrigen Aufwandmengen und Konzentrationen zufriedenstellend.

Es wurden neue Azolylether der allgemeinen Formel 1

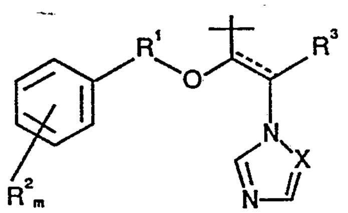

(I)

in der
$R^1$ eine Alkylenkette mit 2 bis 10 Kohlenstoff-Atomen, die durch 1 bis 3 $C_1$- bis $C_4$-Alkylreste substituiert sein kann,
$R^2$ Wasserstoff, $C_1$- bis $C_4$-Alkyl, $C_1$- bis $C_4$-Alkoxy, Halogen, Nitro, Trifluormethyl, halogeniertes $C_1$- bis $C_4$-Alkoxy, Phenoxy oder Benzyloxy,
$R^3$ Wasserstoff, $C_1$- bis $C_4$-Alkyl, $C_1$- bis $C_4$-Alkoxy,
m 1, 2, 3 und
X CH oder N
und die gestrichelte und die durchgezogene Linie eine Einfachbindung oder eine Doppelbindung bedeuten, sowie deren für Pflanzen verträgliche Säureadditionssalze und Metallkomplexe gefunden, die eine sehr gute fungizide Wirkung bei ausgezeichneter Pflanzenverträglichkeit haben.

Der Arylrest kann beispielsweise folgendermassen durch $R^2$ substituiert sein, wobei die 1 bis 3 Reste $R^2$ identisch oder unterschiedlich sein können.

Wasserstoff
2-Methyl
4-Methyl
3-Methyl
3-tert.-Butyl
4-tert.-Butyl
2-Methoxy
3-Methoxy
4-Methoxy
3,5-Dimethoxy
4-tert.-Butoxy
3-Trifluormethyl
4-Trifluormethyl
3-Nitro
4-Nitro
2-Fluor
3-Fluor
4-Fluor
2-Chlor
3-Chlor
4-Chlor
4-Brom
4-Iod
2,4-Dichlor
3,5-Dichlor
2,6-Dichlor
2,4,6-Trichlor
3-Trifluormethoxy
4-Trifluormethoxy
3-Phenoxy
4-Phenoxy
4-Benzyloxy

$R^1$ bedeutet beispielsweise
Propylen, Butylen, Pentylen, Hexylen, Heptylen, Oktylen, 2-Methylpropylen, 3-Methylpentylen, 2-Methylpentylen, 2-Ethylpropylen, 2-Propylpropylen, 2,4-Dimethylpentylen.

$R^3$ kann beispielsweise Wasserstoff, Methyl, Ethyl, Methoxy, Ethoxy, Propoxy, Butoxy sein.

Geeignete Säureadditionssalze sind beispielsweise Bromide, Sulfate, Nitrate, Phosphate, Oxalate, Dodecylsulfonate. Die Wirksamkeit der Salze geht auf das Kation zurück, so dass die Wahl des Anions, sieht man von der Pflanzenverträglichkeit ab, beliebig ist.

Geeignete Metallkomplexe sind Verbindungen der allgemeinen Formel IV,

$$Me\left[(I)_Y\right]Q_X \qquad (IV)$$

in der I die oben angegebene Bedeutung hat,
Me ein Äquivalent eines Metallions, z.B. Kupfer, Zink, Zinn, Mangan, Eisen, Cobalt oder Nickel,
Q ein Äquivalent des Anions einer anorganischen Säure, z.B. Salzsäure, Schwefelsäure, Phosphorsäure oder Bromwasserstoffsäure, bedeutet und
X und Y für die zum Ausgleich der Wertigkeiten erforderlichen Zahlen stehen.

Es wurde ausserdem ein Verfahren zur Herstellung der Azolylether der allgemeinen Formel I, gemäss Anspruch 1 gefunden, das dadurch gekennzeichnet ist, dass man Ketone der allgemeinen Formel IIa oder Alkohole der allgemeinen Formel IIb

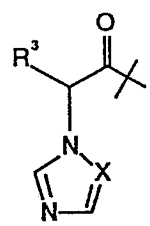

(IIa)

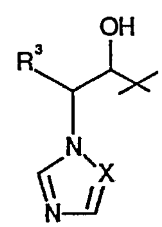

(IIb)

in der $R^3$ und X die angegebenen Bedeutungen haben, oder deren Alkalienolate bzw. Alkoholate mit einem Arylalkylhalogenid, -mesylat oder -tosylat der allgemeinen Formel III

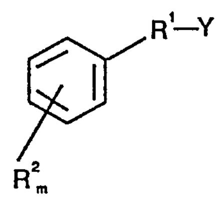

(III)

in der $R^1$, $R^2$ und m die in Anspruch 1 angegebenen Bedeutungen haben und Y für Chlor, Brom

Iod, Mesylat ($O-SO_2CH_3$) oder Tosylat ($O-SO_2-C_6H_4-CH_3$) steht, gegebenenfalls in Gegenwart eines Lösungs- oder Verdünnungsmittels gegebenenfalls in Gegenwart anorganischer oder organischer Basen bei Temperaturen zwischen 0 und 200°C umsetzt.

Das Verfahren zur Herstellung der Azolylether der allgemeinen Formel I besteht beispielsweise darin, dass man Ketone der allgemeinen Formel IIa (DE-OS 2 638 470, DE-OS 2 431 407, DE-OS 3 150 204) bzw. Azolylalkanole der allgemeinen Formel IIb (DE-OS 2 431 407; DE-OS 2 926 280; CA 94, 4020) oder deren Alkalienolate bzw. Alkoholate mit Arylalkylhalogeniden, -mesylaten oder -tosylaten der allgemeinen Formel III, gegebenenfalls in Gegenwart einer Base und/oder eines Lösungs- oder Verdünnungsmittels zu den neuen Azolylethern der allgemeinen Forme I alkyliert.

Die Umsetzung erfolgt beispielsweise in folgender allgemeiner Form.

a)

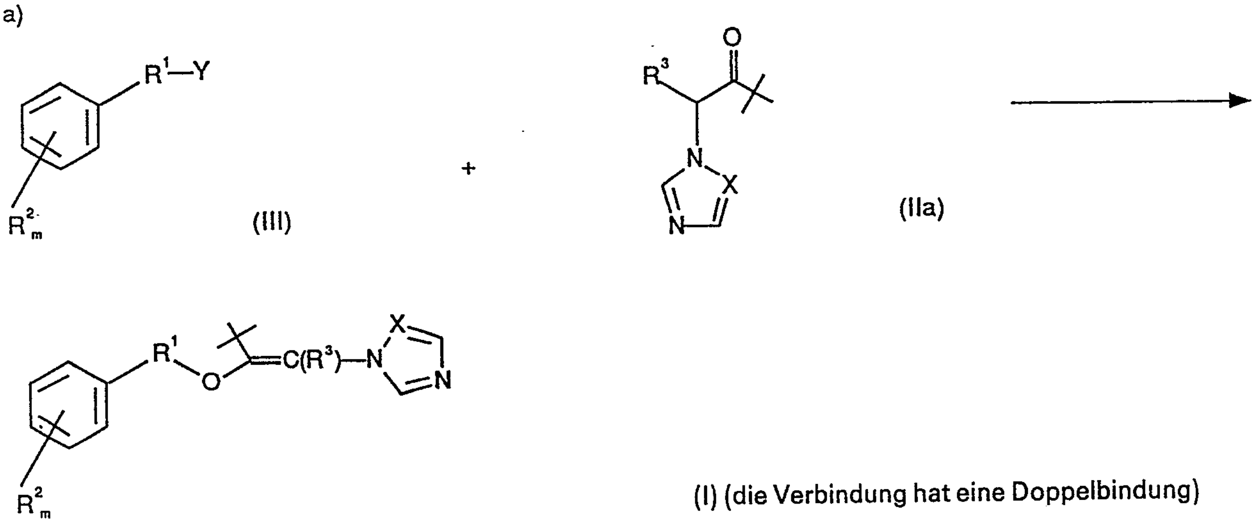

(III) + (IIa)

(I) (die Verbindung hat eine Doppelbindung)

Hierzu können die Ketone IIa zunächst zu den Alkalienolaten metalliert werden, indem man sie vorzugsweise in Gegenwart eines polaren aprotischen Lösungsmittels wie Dimethylformamid oder Tetrahydrofuran mit 0,8 bis 1,2 Äquivalenten, bevorzugt 1,0 Äquivalent, eines Metallierungsreagens wie Natriumhydrid, Lithiumdiisopropylamid oder n-Butyllithium bei 0 bis 200°C, vorzugsweise bie 10 bis 50°C umsetzt. Nach anschliessender Zugabe von 0,8 bis 2,0, bevorzugt 1,0, Äquivalent des jeweiligen Arylalkylhalogenids, -mesylats oder -tosylats, der allgemeinen Formel III erhält man bei Reaktionstemperaturen zwischen 0 und 200°C, bevorzugt bei 30 bis 150°C, die Azolylether der allgemeinen Formel I neben den Ketonen folgender allgemeinen Formel

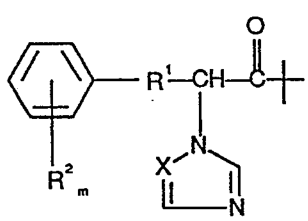

falls $R^3$ Wasserstoff bedeutet.

Eine Variante dieses Verfahrens besteht darin, dass man die Ketone IIa, in Gegenwart von 0,8 bis 1,2; bevorzugt 1,0, Äquivalenten einer Base z.B. Kalium-tert.-butoxid, Natriummethoxid oder Kaliumhydroxid mit den Arylalkylhalogeniden, -mesylaten oder -tosylaten umsetzt, wobei man zweckmässig in Gegenwart eines Lösungs- oder Verdünnungsmittels bei Temperaturen zwischen 0 und 200°C, bevorzugt bei 30 bis 150°C, arbeitet.

Als Lösungsmittel oder Verdünnungsmittel kommen hier wiederum dipolare aprotische Lösungsmittel in Betracht, aber auch Alkohole wie Methanol oder tert.-Butanol.

Verwendet man Alkohole als Ausgangsprodukte dann ergibt sich beispielsweise folgende Umsetzung in allgemeiner Form:

b)

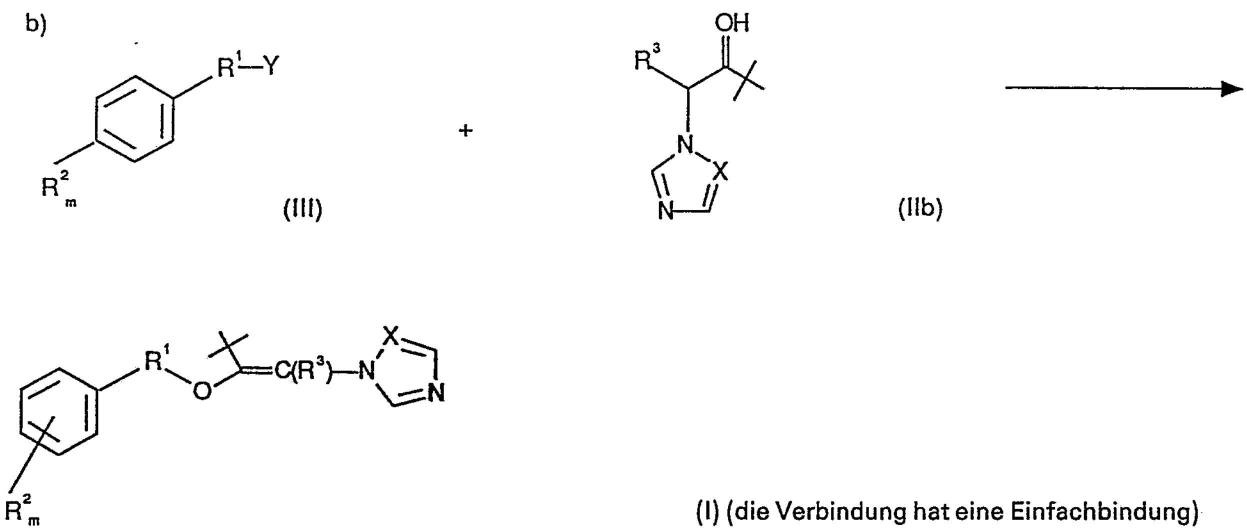

(III) + (IIb)

(I) (die Verbindung hat eine Einfachbindung)

Hierzu können die Alkanole IIb zunächst zu den Alkoholaten metalliert werden, indem man sie vorzugsweise in Gegenwart eines polaren aprotischen Lösungsmittels wie Dimethylformamid oder Tetrahydrofuran mit 0,8 bis 1,2 Äquivalenten, bevorzugt 1,0 Äquivalent, eines Metallierungsreagens wie Natriumhydrid, Lithiumdiisopropylamid oder n-Butyllithium bei 0 bis 200°C, vorzugsweise bei 10 bis 50°C, umsetzt. Nach anschliessender Zugabe von 0,8 bis 2,0, bevorzugt 1,0, Äquivalenten des jeweiligen Arylalkylhalogenids, -mesylats oder -tosylats der allgemeinen Formel II erhält man bei Reaktionstemperaturen zwischen 0 bis 200°C, bevorzugt bei 30 bis 150°C die Azolylether der allgemeinen Formel I.

Eine Variante dieses Verfahrens besteht darin, dass man die Alkohole IIb in Gegenwart von 0,8 bis 1,2, bevorzugt 1,0, Äquivalenten einer Base z.B. Kalium-tert.-butoxid, Natriummethoxid oder Kaliumhydroxid mit den Arylalkylhalogeniden, -mesylaten oder tosylaten umsetzt, wobei man zweckmässigerweise in Gegenwart eines Lösungs- oder Verdünnungsmittels bei Temperaturen zwischen 0 und 200°C, bevorzugt bei 30 bis 150°C, arbeitet.

Zur Isolierung der neuen Verbindungen wird nach üblichen Methoden vorgegangen. Im allgemeinen bedürfen die anfallenden Produkte einer weiteren Reinigung; sie können nach bekannten Methoden wie Umkristallisation, Extraktion, Destillation und Säulenchromatographie gereinigt werden.

Die neuen Verbindungen I können in 2 geometrischen Isomeren (Z und E) und je nach An- bzw. Abwesenheit von Chiralitätszentren in der Alkylenkette $R^1$ bzw. der Beschaffenheit von $R^3$ in Form von Enantiomeren - bzw. Diastereomerengemischen auftreten, die mit üblichen Methoden in die einzelnen Isomeren getrennt werden können. Die einzelnen Isomeren zeigen unterschiedliche biologische Aktivität; sie sind ebenso wie die Isomerengemische Gegenstand der Erfindung, d.h. die reinen Diastereomeren und die reinen Enantiomeren sind ebenso wie deren Gemische Bestandteil der Erfindung. Für den Einsatz als Fungizide genügt die Verwendung der Gemische, wenn auch in gewissen Fällen die Verwendung von reinen Isomeren eine Reduzierung der Aufwandmenge ermöglicht.

Falls gewünscht, können die neuen Verbindungen der allgemeinen Formel I auch in pflanzenverträgliche Salze mit anorganischen oder organischen Säuren übergeführt werden, wie beispielsweise in Salze der Salzsäure, Bromwasserstoffsäure, Salpetersäure, Oxalsäure, Essigsäure, Schwefelsäure, Phosphorsäure oder Dodecylbezolsulfonsäure. Die Wirksamkeit der Salze geht auf das Kation zurück, so dass die Wahl des Anions beliebig ist.

Ferner lassen sich die Verbindungen der allgemeinen Formel I nach bekannten Methoden in Metallkomplexe überführen. Das kann durch Umsetzung dieser Verbindungen mit geeigneten Metallsalzen wie beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Die Herstellung der neuen Verbindungen der allgemeinen Formel I wird durch folgende Beispiele erläutert:

Beispiel 1

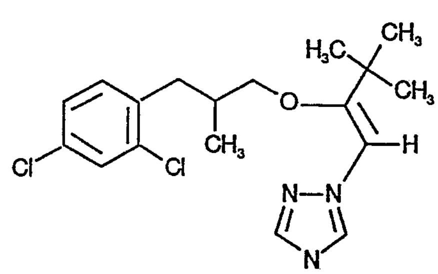

2-(gamma-2,4-Dichlorphenyl-β-methylpropoxy)-3,3-dimethyl-1-(1,2,4-triazol-1-yl)-1-buten

Zu einer Suspension von 28,8 g (1,2 mol) Natriumhydrid in 150 ml Dimethylformamid wird bei 20°C eine Lösung von 167 g (1,0 mol) 3,3-Dimethyl-1-(1,2,4-triazol-1-yl)-butan-2-on gelöst in 250 ml Dimethylformamid zugetropft. Nach 2 Stunden Rühren bei 70°C werden 237,5 g (1,0 mol) 1-(2,4-Dichlorphenyl)-2-methyl-3-chlorpropan zugetropft. Man rührt 5 Stunden bei Rückflusstemperatur, lässt danach erkalten, tropft 300 ml Wasser zu und extrahiert mit Methylenchlorid.

Die organische Phase wird mit Natriumsulfat getrocknet und eingeengt. Das zurückbleibende Öl wird im Vakuum destilliert. Die bei 190 bis 192°C 0,005 mbar übergehende Fraktion wird gesammelt. 73,6 g davon werden mittels 9,3 g Natriumborhydrid in Methanol reduziert. Hierdurch werden unerwünschte Nebenprodukte reduziert. Nach Einengen des Lösungsmittels wird der Rückstand mit Wasser und Ether extrahiert. Die organische Phase wird über Natriumsulfat getrocknet und mittels Chromatographie an Silika-Gel mit Cyclohexan-Essigester die Nebenprodukte abgetrennt.

Auf diese Weise erhält man in 28-%iger Ausbeute reines Produkt, dessen Struktur durch spektroskopische Daten belegt wird (Verbindung Nr. 1).

$^1$H-NMR ($CDC1_3$, 20o MHZ-Spektrum, ppm)

| | |
|---|---|
| 8,25 (1H) s | } Triazol |
| 7,98 (1H) s | |
| 7,0–7,27 (3H) | Aromat |
| 6,3 (1H) s | |
| 3,2 (2H) d | |
| 2,8 (1H) mc | |
| 2,4 (1H) mc | |
| 2,1 (1H) mc | |
| 1,2 (9H) s | tert.-Butyl |
| 0,85 (3H) d | Methyl |

s Singulett
d Duplett
mc Zentrum eines Multipletts

Massenspektrum (T 250°C)

367 ($M,^+$)

167 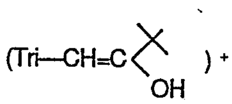

159, 161 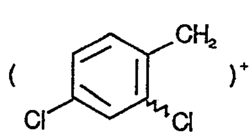

90,52 MHZ-C-NMR-Spektrum

| | |
|---|---|
| 16,40 | 126,9; 129,4; 131,9; 132,6; |
| 27,92 | 134,87; 136,4. |
| 36, 86; 26,80; 34, 53; | 151,68; 145,0. |
| 76,07; | |
| 104, 33; | |

Beispiel 2

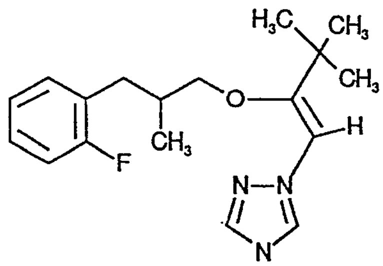

2-(gamma-2-Fluorphenyl-β-methylpropoxy)-3,3-dimethyl-1-(1,2,4-triazol-1-yl)-1-buten

In einer Suspension von 8,18 g (0,341 mol) Natriumhydrid in 50 ml Dimethylformamid wird bei 20°C eine Lösung von 47,4 g (0,284 mol) 3,3-Dimethyl-1-(1,2,4-triazol-1-yl)-butan-2-on gelöst in 70 ml Dimethylformamid zugetropft. Nach 2 Stunden Rühren bei 60°C wird 53 g (0,284 mol) 1-(2-Fluorphenyl)-2-methyl-3-chlorpropan zugetropft. Man rührt 5 Stunden bei Rückflusstemperatur, lässt hernach erkalten, tropft 300 ml Wasser zu und extrahiert mit Methylenchlorid.

Die organische Phase wird mit Natriumsulfat getrocknet und eingeengt. Das zurückbleibende Öl wird im Vakuum destilliert. Die bei 161°C/∅,001 mbar übergehende Fraktion wird gesammelt. 20 g davon werden mittels 2,93 g Natriumborhydrid in 350 ml Methanol reduziert. Hierdurch werden unerwünschte Nebenprodukte reduziert.

Nach Einengen des Lösungsmittels wird der Rückstand mit Wasser und Ether extrahiert. Die organische Phase wird über Natriumsulfat getrocknet und mittels Chromatographie an Silika-Gel mit Cyclohexan-Essigester getrennt.

Auf diese Weise erhält man 3 g reines Produkt, dessen Struktur durch spektroskopische Daten belegt wird (Verbindung Nr. 2).

$^1$H-NMR ($CDCl_3$, 200 MHZ-Spektrum, ppm)

| | |
|---|---|
| 8,25 (s, 1H) | } Triazol |
| 7,98 (s, 1H) | |
| 7,05 (mc, 4) | Aromat |
| 6,3 (s, 1H) | |
| 1,2 (s, 9H) | tert.-Butyl |
| 0,82 (s, 3H) | } Methyl |
| 0,88 (s | |

s Singulett
d Duplett
mc Zentrum eines Multipletts

Beispiel 3

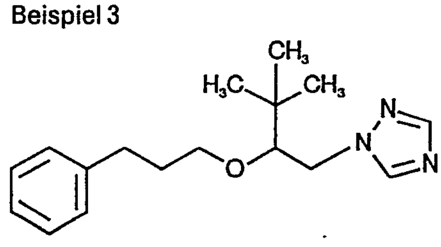

Zu einer Lösung von 40 g 3,3-Dimethyl-1,1-(1,2,4-triazol-1-yl)-butan-2-ol und 60 g 3-Phenylpropylbromid in 390 ml Ether und 110 ml Dimethylsulfoxid werden 18 g NaH (80%ige Paraffinsuspension) portionsweise zugegeben. Nach Beendigung der $H_2$-Entwicklung wird 30 min. zum Rückfluss erwärmt. Nach Abkühlen wird nach Zugabe von 1,5 1 $H_2O$ mit Ether extrahiert. Die organische Phase wird mehrmals mit Wasser gewaschen, über $Na_2SO_4$ getrocknet, eingeengt und destilliert.

Man erhält 26 g 3,3-Dimethyl-2-(3-phenylpropoxy)-1-(1,2,4-triazol-1-yl)-butan. Sdp. 159 bis 160°C/0,3 mbar (Verbindung 3).

Beispiel 4

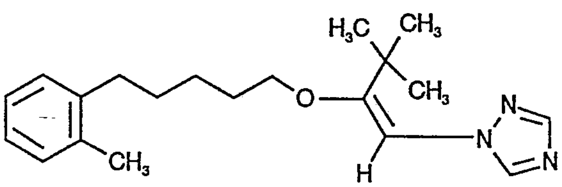

2-(epsilon-2-Methylphenylpentyloxy)-3,3-dimethyl-1-(1,2,4-triazol-1-yl)-1-buten

Zu einer Suspension von 9,18 g (0,38 mol) Natriumhydrid in 50 ml Dimethylformamid wird bei 20°C eine Lösung von 51,9 g (0,31 mol) 3,3-Dimethyl-1-(1,2,4-triazol-1-yl)-butan-2-on gelöst in 100 ml Dimethylformamid zugetropft. Nach 2 Stunden Rühren bei 70°C wird 62,7 g (0,31 mol) 1-(2-Methylphenyl)-5-chlorpentan zugetropft. Nach Rühren während 5 Stunden bei Rückflusstemperatur und anschliessendem Abkühlen auf Raumtemperatur tropft man 150 ml Wasser zu und extrahiert mit Methylchlorid. Die organische Phase wird über Natriumsulfat getrocknet und eingeengt. Das zurückbleibende Öl wird im Vakuum destilliert; die bei 204 bis 206°C/0,01 mbar übergehende Fraktion wird gesammelt (54,5 g).

Elementaranalyse:

| | C | H | N |
|---|---|---|---|
| Ber.: | 73,4 | 8,9 | 12,8% |
| Gef.: | 73,5 | 9,1 | 12,8%. |

21,5 g davon werden mit 3,0 g Natriumborhydrid in 300 ml Methanol reduziert. Hierdurch werden unerwünschte Nebenprodukte reduziert.

Nach Einengen des Lösungsmittels wird der Rückstand mit Wasser und Ether extrahiert.

Die organische Phase wird über Natriumsulfat getrocknet und mittels Säulenchromatographie an Silika-Gel mit Cyclohexan-Essigester getrennt.

Auf diese Weise erhält man 6,9 g reines Produkt, dessen Struktur durch spektroskopische Daten belegt wird (Verbindung Nr. 4).

$^1$H-NMR ($CDCl_3$, 200 MHZ, ppm)

8,32 (s, 1H)
7,95 (s, 1H)
7,05 (s, 4H)
6,3 (s, 1H)
3,35 (m, 2H)
2,58 (m, 2H)
2,3 (s, 3H)
1,20 (s, 9H)

s Singulett
m Multiplett

$^{13}$-C-Spektrum ($CDCl_3$, 90. 52 MHZ)

| | | |
|---|---|---|
| 140,55 | 33,15 | 163,52 |
| 135,75 | 30,02 | 36,72 |
| 160,16 | 29,96 | 27,89 |
| 178,89 | 71,92 | 105,30 |
| 125,88 | | 151,53 |
| 19,6 | | 144,78 |

Entsprechend können folgende Verbindungen hergestellt werden:

Wirkstoffliste

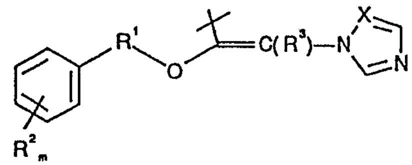

| Verbindung Nr. | $R^1$ | $R^2_m$ | $R^3$ | X | Triazol | Aromat | tert.-Butyl | $^1$HNMR-Daten (ppm) $R^3 = H$ |
|---|---|---|---|---|---|---|---|---|
| 5 | $-CH_2-CHCH_3-CH_2-$ | 4-$CH_3$ | H | N | 8,25;7,95 | 7,0 (AA'BB') | 1,2 | 6,28 |
| 6 | $-CH_2-CHCH_3-CH_2-$ | 4-tBu | H | N | 8,28;7,98 | 7,2 (AA'BB') | 1,2 (1,36) | 6,3 |
| 7 | $-CH_2-CHCH_3-CH_2-$ | 4-F | H | N | 8,25;7,98 | 6,99 | 1,2 | 6,28 |
| 8 | $-CH_2-CHCH_3-CH_2-$ | 4-$OCH_3$ | H | N | 8,28;7,98 | 6,9 (AA'BB') | 1,2 | 6,3 |
| 9 | $-(CH_2)_4-$ | H | H | N | – | – | 1,2 | 6,35 |
| 10 | $-(CH_2)_5-$ | 4-$CH_3$ | H | N | 8,3;7,97 | 7,05 | 1,2 | 6,3 |
| 11 | $-(CH_2)_5-$ | 4-Cl | H | N | 8,32;8,0 | 7,15 (AA'BB') | 1,12 | 6,35 |
| 12 | $-(CH_2)_5-$ | H | H | N | 8,32;7,98 | 7,22 | 1,2 | 6,36 |
| 13 | $-(CH_2)_6-$ | H | H | N | 8,32;8,0 | 7,2 | 1,2 | 6,34 |
| 14 | $-(CH_2)_7-$ | H | H | N | 8,35;8,0 | 7,2 | 1,23 | 6,37 |
| 15 | $-CH_2-CH_2-CHCH_3-CH_2-CH_2-$ | H | H | N | 8,32;8,0 | 7,2 | 1,24 | 6,35 |
| 16 | $-CH_2-CH_2-CH_2-CHCH_3-CH_2-$ | H | H | N | 8,28;7,98 | 7,17 | 1,18 | 6,32 |
| 17 | $-CH_2-CHCH_3-CH_2-$ | 2,4-$Cl_2$ | $OCH_3$ | N | 8,26;8,0 | 6,9–7,4 | 1,2 | 3,3 ($R^3 = OCH_3$) |

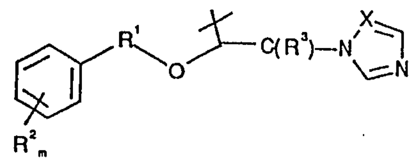

| Verbindung Nr. | $R^1$ | $R^2_m$ | $R^3$ | X | Sdp. |
|---|---|---|---|---|---|
| 18 | $-CH_2-CHCH_3-CH_2-$ | H | H | N | |
| 19 | $-(CH_2)_4-$ | H | H | N | 170–178 °C, 0,2 mbar |
| 20 | $-(CH_2)_5-$ | H | H | N | |
| 21 | $-CH_2-CHCH_3-CH_2-$ | 4-tBu | H | N | |
| 22 | $-CH_2CHCH_3CH_2-$ | 4-Cl | H | N | |
| 23 | $-CH_2CHCH_3CH_2-$ | 2,4-$Cl_2$ | H | N | |
| 24 | $-(CH_2)_3-$ | H | H | CH | |
| 25 | $-(CH_2)_4-$ | H | H | CH | |
| 26 | $-(CH_2)_3-$ | H | $CH_3$ | N | |
| 27 | $-(CH_2)_3-$ | H | $OCH_3$ | N | 166–175 °C, 0,2 mbar |
| 28 | $-(CH_2)_3-$ | H | $O(CH_2)_2CH_2$ | N | 165–172 °C, 0,1 mbar |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungizden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse – wie Gurken, Bohnen und Kürbisgewächse –.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten,
Verticillum-Arten in Baumwolle und Gemüse.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden,wie Lösungen, Emulsionen, Suspensionen, Stäube, Pulver, Pasten und Granulate. Die Anwendungsformen richten sich ganz nach den Verwendungszwekken; sie sollen in jedem Fall eine feine und gleichmässige Verteilung der wirksamen Substanz gewährleisten. Die Formulierungen werden in bekannter Weise hergestellt, z.B. durch Verstrekken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, dass man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Beispiele für solche Zubereitungen sind:

1. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

2. 20 Gewichtsteile der Verbindung Nr. 11 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in Wasser erhält man eine wässrige Dispersion.

3. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und feines Verteilen der Lösung in Wasser erhält man eine wässrige Dispersion.

4. 20 Gewichtsteile der Verbindung Nr. 6 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingiessen und feines Verteilen der Lösung in Wasser erhält man eine wässrige Dispersion.

5. 80 Gewichtsteile der Verbindung Nr. 1 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

6. 3 Gewichtsteile der Verbindung Nr. 12 werden mit 97 Gewichtsteilen feinteiligem Kaolin innig vermischt. Man erhält auf diese Weise ein Stäubemittel, das 3 Gew.% des Wirkstoffs enthält.

7. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

8. 40 Gewichtsteile der Verbindung Nr. 5 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wässrige Dispersion. Durch Verdünnen mit Wasser erhält man eine wässrige Dispersion.

9. 20 Teile der Verbindung Nr. 6 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd Kondensates und 68 Teilen eines paraffinischen Mineralöls innig vermischt. Man erhält eine stabile ölige Dispersion.

Die erfindungsgemässen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrösserung des fungiziden Wirkungsspektrums.

Die folgende Liste von Fungiziden, mit denen die erfindungsgemässen Verbindungen kombiniert werden können, soll die Kombinationsmöglichkeiten erläutern, nicht aber einschränken.

Fungizide, die mit den neuen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Magan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat) Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäureanilid
2-Methyl-furan-3-carbonsäureanilid

2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-lod-benzoesäure-anilid
N-Formyl-N-morpholin-2,2,2-trichlorethylacetal
Piperazin-1,4-diylbis-(1-(2,2,2-trichlorethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazolyl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methylpyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie Dodecylguanidinacetat
3-(3-(3,5-Dimethyl-2-oxycyclohexyl)-2-hydroxyethyl)-gluataramid Hexachlorbenzol
DL-Methyl-N-(2,6-dimethylphenyl)-N-furoyl(2)-alaninat,
DL-N-(2,6-Dimethylphenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-N-(ethylaminocarbonyl)-2-methoximino-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol

Für die folgenden Versuche wurde als Vergleichswirkstoff die bekannte Verbindung 1-(2'-(2,4-Dichlorphenyl)-2'-(2-propenyloxy)-ethyl)-1H-imidazol (A) verwendet.

Versuch 1
Wirksamkeit gegen Weizenmehltau

Blätter von in Töpfen gewachsenen Weizenkeimlingen der Sorte «Frühgold» werden mit wässriger Spritzbrühe, die 80% Wirkstoff und 20% Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschliessend im Gewächshaus bei Temperaturen zwischen 20 und 22°C und 75 bis 80% relativer Luftfeuchtigkeit aufgestellt. Nach 7 Tagen wird das Ausmass der Mehltauentwicklung ermittelt.

Das Ergebnis des Versuches zeigt, dass die Verbindungen 1, 6 und 11 bei der Anwendung als 0,006 und 0,0015%ige Wirkstoffbrühen eine bessere fungizide Wirkung (z.B. 97%) zeigen als der Wirkstoff A (z.B. 70%).

Versuch 2
Wirksamkeit gegen Gurkenmehltau

Blätter von in Töpfen gewachsenen Gurkenkeimlingen der Sorte «Chinesische Schlange» werden im Zweiblattstadium mit einer Sporensuspension des Gurkenmehltaus (Erysiphe cichoracearum) besprüht. Nach etwa 20 Stunden werden die Versuchspflanzen mit wässriger Spritzbrühe, die 80% Wirkstoff und 20% Emulgiermittel in der Trockensubstanz enthält, bis zur Tropfnässe besprüht. Nach dem Antrocknen des Spritzbelages werden sie anschliessend im Gewächshaus bei Temperaturen zwischen 20 und 22°C und 70 bis 80% relativer Luftfeuchtigkeit aufgestellt. Zur Beurteilung der Wirksamkeit der neuen Stoffe wird das Ausmass der Pilzentwicklung nach 21 Tagen ermittelt.

Das Ergebnis des Versuchs zeigt, dass die Wirkstoffe 1, 6 und 12 bei der Anwendung als 0,025%ige Wirkstoffbrühe eine bessere fungizide Wirkung (z.B. 100%) zeigen als der Wirkstoff A (z.B. 70%).

Versuch 3
Wirksamkeit gegen Pyricularia oryzae (protektiv)

Blätter von in Töpfen gewachsenen Reiskeimlingen der Sorte «Bahia» werden mit wässrigen Emulsionen, die 80% Wirkstoff und 20% Emulgiermittel in der Trockensubstanz enthalten, tropfnass besprüht und 24 Stunden später mit einer wässrigen Sporensuspension von Pyricularia oryzae inoculiert. Anschliessend werden die Versuchspflanzen in Klimakammern bei 22 bis 24°C und 95 bis 99% relativer Luftfeuchtigkeit aufgestellt. Nach 6 Tagen wird das Ausmass des Krankheitsbefalls ermittelt.

Das Ergebnis des Versuches zeigt, dass die Wirkstoffe 1, 5, 7, 8, 9, 10, 11, 13, 15 und 16 bei der Anwendung als 0,05%ige Wirkstoffbrühe eine gute fungizide Wirkung (z.B. 97%) zeigen.

**Patentansprüche für die Vertragsstaaten BE, CH, DE, FR, GB, IT, LI, NL, SE**

1. Azolylether der allgemeinen Formel I

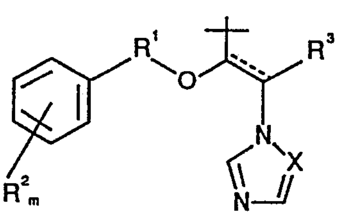

(I)

in der

$R^1$ eine Alkylenkette mit 2 bis 10 Kohlenstoffatomen, die durch 1 bis 3 $C_1$- bis $C_4$-Alkylreste substituiert sein kann,
$R^2$ Wasserstoff, $C_1$- bis $C_4$-Alkyl, $C_1$- bis $C_4$-Alkoxy,

Halogen, Nitro, Trifluormethyl, halogeniertes $C_1$- bis $C_4$-Alkoxy, Phenoxy oder Benzyloxy,
$R^3$ Wasserstoff, $C_1$- bis $C_4$-Alkyl, $C_1$- bis $C_4$-Alkoxy,
m 1, 2, 3 und
X CH oder N
und die gestrichelte und die durchgezogene Linie eine Einfachbindung oder eine Doppelbindung bedeuten, sowie deren für Pflanzen verträgliche Säureadditionssalze und Metallkomplexe.

2. Verfahren zur Herstellung der Azolylether der allgemeinen Formel I gemäss Anspruch 1, dadurch gekennzeichnet, dass man Ketone der allgemeinen Formel IIa oder Alkanole der allgemeinen Formel IIb

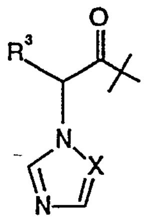 (IIa) 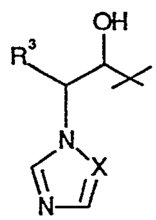 (IIb)

worin $R^3$ und X die in Anspruch 1 angeführte Bedeutung haben, oder deren Alkalienolate bzw. Alkoholate mit Arylalkylhalogeniden, -mesylaten oder -tosylaten der allgemeinen Formel III

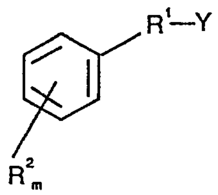 (III)

in der $R^1$ und $R^2$ und m die in Anspruch 1 angegebenen Bedeutungen haben und Y Chlor, Brom, Iod, Mesylat ($O\text{-}SO_2\text{-}CH_3$) oder Tosylat

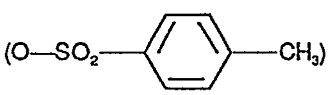

bedeuten, gegebenenfalls in Gegenwart eines Lösungs- oder Verdünnungsmittels und gegebenenfalls in Gegenwart anorganischer oder organischer Basen bei Temperaturen zwischen 0 und 200°C umsetzt.

3. Fungizide Mittel, enthaltend eine Verbindung gemäss Anspruch 1.

4. Fungizide Mittel, enthaltend eine Verbindung gemäss Anspruch 1 und einen festen oder flüssigen Trägerstoff.

5. Verfahren zur Herstellung von Fungiziden, dadurch gekennzeichnet, dass man eine Verbindung der allgemeinen Formel I gemäss Anspruch 1 mit einem festen oder flüssigen Trägerstoff mischt.

6. Verfahren zur Bekämpfung von Fungi, dadurch gekennzeichnet, dass man eine Verbindung der allgemeinen Formel I gemäss Anspruch 1 auf diese einwirken lässt.

7. Verfahren zur vorbeugenden Bekämpfung von Fungi, dadurch gekennzeichnet, dass man eine Verbindung der allgemeinen Formel I gemäss Anspruch 1 auf durch Pilzbefall bedrohte Matererialien, Flächen, Pflanzen oder Saatgüter einwirken lässt.

**Patentansprüche für den Vertragsstaat AT**

1. Fungizid, enthaltend einen Azolylether der allgemeinen Formel I

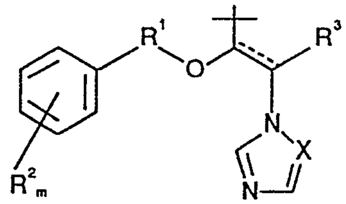 (I)

in der
$R^1$ eine Alkylenkette mit 2 bis 10 Kohlenstoffatomen, die durch 1 bis 3 $C_3$- bis $C_4$-Alkylreste substituiert sein kann,
$R^2$ Wasserstoff, $C_1$- bis $C_4$-Alkyl, $C_1$- bis $C_4$-Alkoky, Halogen, Nitro, Trifluormethyl, halogeniertes $C_1$- bis $C_4$-Alkoxy, Phenoxy oder Benzyloxy,
$R^3$ Wasserstoff, $C_1$- bis $C_4$-Alkyl, $C_1$- bis $C_4$-Alkoxy,
m 1, 2, 3 und
X CH oder N
und die gestrichelte und die durchgezogene Linie eine Einfachbindung oder eine Doppelbindung bedeuten oder dessen für Pflanzen verträgliches Säureadditionssalz doer Metallkomplex.

2. Verfahren zur Herstellung der Azolylether der allgemeinen Formel I gemäss Anspruch 1, dadurch gekennzeichnet, dass man Ketone der allgemeinen Formel IIa oder Alkanole der allgemeinen Formel IIb

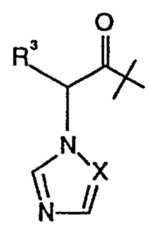 (IIa) 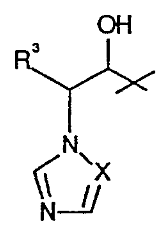 (IIb)

worin $R^3$ und X die in Anspruch 1 angeführte Bedeutung haben, oder deren Alkalienolate bzw. Alkoholate mit Arylalkylhalogeniden, -mesylaten oder -tosylaten der allgemeinen Formel III

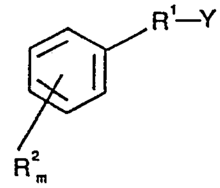 (III)

in der $R^1$ und $R^2$ und m die in Anspruch 1 angegebenen Bedeutungen haben und Y Chlor, Brom, Iod, Mesylat ($O\text{-}SO_2\text{-}CH_3$) oder Tosylat ($O\text{-}SO_2$-

$C_6H_4$-$CH_3$) bedeuten, gegebenenfalls in Gegenwart eines Lösungs- oder Verdünnungsmittels und gegebenenfalls in Gegenwart anorganischer oder organischer Basen bei Temperaturen zwischen 0 und 200°C umsetzt.

3. Fungizide Mittel, enthaltend eine Verbindung gemäss Anspruch 1 und einen festen oder flüssigen Trägerstoff.

4. Verfahren zur Herstellung von Fungiziden, dadurch gekennzeichnet, dass man eine Verbindung der allgemeinen Formel I gemäss Anspruch 1 mit einem festen oder flüssigen Trägerstoff mischt.

5. Verfahren zur Bekämpfung von Fungi, dadurch gekennzeichnet, dass man eine Verbindung der allgemeinen Formel I gemäss Anspruch 1 auf diese einwirken lässt.

6. Verfahren zur vorbeugenden Bekämpfung von Fungi, dadurch gekennzeichnet, dass man eine Verbindung der allgemeinen Formel I gemäss Anspruch 1 auf durch Pilzbefall bedrohte Materialien, Flächen, Pflanzen oder Saatgüter einwirken lässt.

**Claims for the Contracting States BE, CH, DE, FR, GB, IT, LI, NL, SE**

1. An azolyl ether of the general formula I

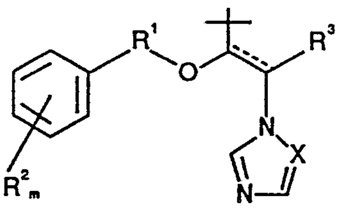

(I)

where
$R^1$ is an alkylene chain of 2 to 10 carbon atoms which may be substituted by from 1 to 3 $C_1$–$C_4$-alkyl radicals,
$R^2$ is hydrogen, $C_1$–$C_4$-alkyl, $C_1$–$C_4$-alkoxy, halogen, nitro, trifluoromethyl, halo-$C_1$–$C_4$-alkoxy, phenoxy or benzyloxy,
$R^3$ is hydrogen, $C_1$–$C_4$-alkyl or $C_1$–$C_4$-alkoxy,
m is 1, 2 or 3 and
X is CH or N.
and the dashed and the solid line are a single bond or a double bond, or a plant-tolerated acid addition salt or metal complex thereof.

2. A process for the preparation of an azolyl ether of the general formula I as claimed in claim 1, wherein a ketone of the general formula IIa or an alkanol of the general formula IIb

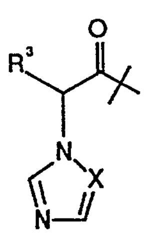

(IIa)

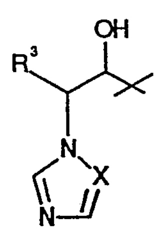

(IIb)

where $R^3$ and X have the meanings stated in claim 1, or its alkali metal enolate or alcoholate, is reacted with an aralalkyl halide, mesylate or tosylate of the general formula III

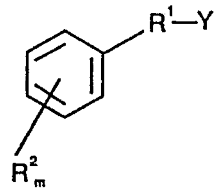

(III)

where $R^1$ and $R^2$ and m have the meanings stated in claim 1 and Y is chlorine, bromine, iodine, mesylate (O-$SO_2$-$CH_3$) or tosylate

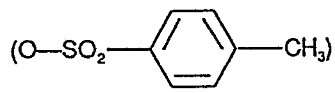

in the presence or absence of a solvent or diluent and in the presence or absence of an inorganic or organic base, at from 0 to 200°C.

3. A fungicide containing a compound as claimed in claim 1.

4. A fungicide containing a compound as claimed in claim 1 and a solid or liquid carrier.

5. A process for the preparation of a fungicide, wherein a compound of the general formula I as claimed in claim 1 is mixed with a solid or liquid carrier.

6. A process for controlling fungi, wherein a compound of the general formula I as claimed in claim 1 is allowed to act on the fungi.

7. A process for the preventive control of fungi, wherein a compound of the general formula I as claimed in claim 1 is allowed to act on materials, surfaces, plants or seed threatened by fungal attack.

**Claims for the Contracting State AT**

1. A fungicide containing an azolyl ether of the general formula I

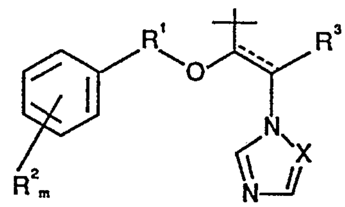

(I)

where
$R^1$ is an alkylene chain of 2 to 10 carbon atoms which may be substituted by from 1 to 3 $C_1$–$C_4$-alkyl radicals,
$R^2$ is hydrogen, $C_1$–$C_4$-alkyl, $C_1$–$C_4$-alkoxy, halogen, nitro, trifluoromethyl, halo-$C_1$–$C_4$-alkoxy, phenoxy or benzyloxy,
$R^3$ is hydrogen, $C_1$–$C_4$-alkyl or $C_1$–$C_4$-alkoxy,
m is 1, 2 or 3 and
X is CH or N.
and the dashed and the solid line are a single

bond or a double bond, or a plant-tolerated acid addition salt or metal complex thereof.

2. A process for the preparation of an azolyl ether of the general formula I as claimed in claim 1, wherein a ketone of the general formula IIa or an alkanol of the general formula IIb

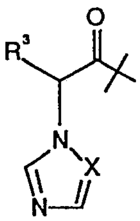
(IIa)
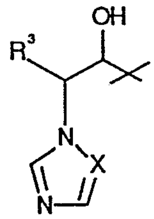
(IIb)

where $R^3$ and X have the meanings stated in claim 1, or its alkali metal enolate or alcoholate, is reacted with an aralalkyl halide,mesylate or tosylate of the general formula III

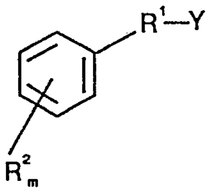
(III)

where $R^1$ and $R^2$ and m have the meanings stated in claim 1 and Y is chlorine, bromine, iodine, mesylate ($O-SO_2-CH_3$) or tosylate

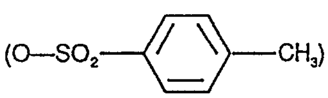

in the presence or absence of a solvent or diluent and in the presence or absence of an inorganic or organic base, at from 0 to 200°C.

3. A fungicide containing a compound as claimed in claim 1 and a solid or liquid carrier.

4. A process for the preparation of a fungicide, wherein a compound of the general formula I as claimed in claim 1 is mixed with a solid or liquid carrier.

5. A process for controlling fungi, wherein a compound of the general formula I as claimed in clam 1 is allowed to act on the fungi.

6. A process for the preventive control of fungi, wherein a compound of the general formula I as claimed in claim 1 is allowed to act on materials, surfaces, plants or seed threatened by fungal attack.

**Revendications pour les Etats contractants: BE, CH, DE, FR, GB, IT, LI, NL, SE**

1. Azolylether de formule générale

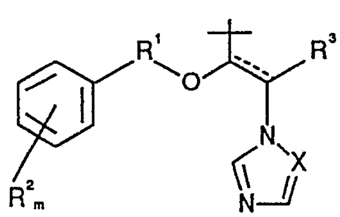
(I)

dans laquelle

$R^1$ représente une chaîne alkylène de 2 à 10 atomes de carbone, qui peut être substituée par 1 à 3 restes alkyle en $C_1$ à $C_4$,

$R^2$ représente hydrogène, alkyle en $C_1$ à $C_4$, alcoxy en $C_1$ à $C_4$, halogène, nitro, trifluorométhyle, alcoxy en $C_1$ à $C_4$ halogéné, phénoxy ou benzyloxy,

$R^3$ hydrogène, alkyle en $C_1$ à $C_4$, alcoxy en $C_1$ à $C_4$,

m 1, 2, 3 et

X CH ou N

et les lignes en pointillé et les lignes continues représentent une liaison simple ou une liaison double, ainsi que leurs sels d'addition d'acide et complexes métalliques acceptable pour les plantes.

2. Procédé de préparation de l'azolylether de formule I selon la revendication 1, caractérisé par le fait que l'on fait réagir, à des températures comprises entre 0 et 200°C, éventuellement en présence d'un solvant ou diluant et éventuellement en présence de bases inorganiques ou organiques, des cétones de formule générale $II_a$ ou des alcanols de formule générale $II_b$

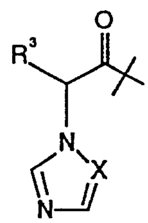
(IIa)
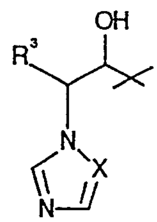
(IIb)

où $R^3$ et X ont les significations indiquées dans la revendication 1, ou leurs énolates alcalins ou alcoolates, avec des halogénures, mesylates ou tosylates d'arylalkyle, de formule générale III

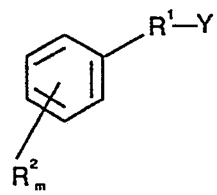
(III)

dans laquelle $R^1$ et $R^2$ et m ont les significations indiquées dans la revendication 1 et Y représente chlore, brome, iode, mesylate ($O-SO_2-CH_3$) ou tosylate

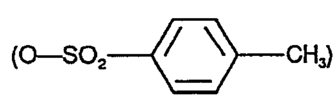

3. Agent fongicide, contenant un composé selon la revendication 1.

4. Agent fongicide contenant un composé selon la revendication 1 et un support solide ou liquide.

5. Procédé de préparation de fongicides, caractérisé par le fait qu'on mélange un composé de formule I selon la revendication 1 avec un support solide ou liquide.

6. Procédé de lutte contre les champignons, caractérisé par le fait que l'on fait agir sur ceux-ci un composé de formule I selon la revendication 1.

7. Procédé de lutte préventive contre les champignons, caractérisé par le fait que l'on fait agir un composé de formule générale I selon la revendication 1 sur des matériaux, surfaces, plantes ou semences menacés par une attaque par des champignons.

**Revendications pour l'Etat Contractant: AT**

1. Fongicide, contenant un azolylether de formule générale

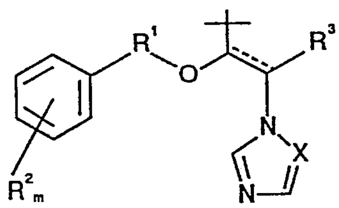

(I)

dans laquelle
$R^1$ représente une chaîne alkylène de 2 à 10 atomes de carbone, qui peut être substituée par 1 à 3 restes alkyle en $C_1$ à $C_4$,
$R^2$ représente hydrogène, alkyle en $C_1$ à $C_4$, alcoxy en $C_1$ à $C_4$, halogène, nitro, trifluorométhyle, alcoxy en $C_1$ à $C_4$ halogéné, phénox ou benzyloxy,
$R^3$ hydrogène, alkyle en $C_1$ à $C_4$, alcoxy en $C_1$ à $C_4$,
m 1, 2, 3 et
X CH ou N
et les lignes en pointillé et les lignes continues représentent une liaison simple ou une liaison double, ainsi que leurs sels d'addition d'acide et complexes métalliques acceptable pour les plantes.

2. Procédé de préparation de l'azolylether de formule I selon la revendication 1, caractérisé par le fait que l'on fait réagir, à des températures comprises ente 0 et 200°C, éventuellement en présence d'un solvant ou diluant et éventuellement en présence de bases inorganiques ou organiques, des cétones de formule générale $II_a$ ou des alcanols de formule générale $II_b$

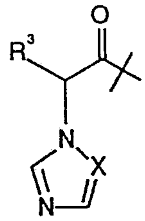

(IIa)

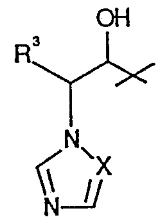

(IIb)

où $R^3$ et X ont les significations indiquées dans la revendication 1, ou leurs énolates alcalins ou alcoolates, avec des halogénures, mesylates ou tosylates d'arylalkyle, de formule générale III

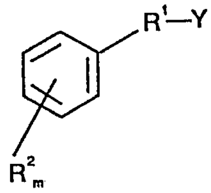

(III)

dans laquelle $R^1$ et $R^2$ et m ont les significations indiquées dans la revendications 1 et Y représente chlore, brome, iode, mesylate ($O-SO_2-CH_3$) ou tosylate ($O-SO_2-C_6H_7-CH_3$).

3. Agent fongicide contenant un composé selon la revendication 1 et un support solide ou liquide.

4. Procédé de préparation de fongicides, caractérisé par le fait qu'on mélange un composé de formule I selon la revendication 1 avec un support solide ou liquide.

5. Procédé de lutte contre les champignons, caractérisé par le fait que l'on fait agir sur ceux-ci un composé de formule I selon la revendication 1.

6. Procédé de lutte préventive contre les champignons, caractérisé par le fait que l'on fait agir un composé de formule générale I selon la revendication 1 sur des matériaux, surfaces, plantes ou semences menacés par une attaque par des champignons.